# EUROPEAN PATENT SPECIFICATION

(11) **EP 2 356 965 B1**
(45) Date of publication and mention of the grant of the patent: **24.07.2013**
(21) Application number: 10157528.0
(22) Date of filing: 24.03.2010
(51) Int. Cl.: A61K 8/02, A61K 8/46, A61Q 9/04

(54) **Method of depilation**
Enthaarungsverfahren
Procédé d'épilation

(30) Priority: 17.02.2010 US 305265 P
(43) Date of publication of application: 17.08.2011
(73) Proprietor: The Procter & Gamble Company, Cincinnati, OH 45202 (US)
(72) Inventor: Smith, Paul, James, Whitton, Middlesex TW2 6EB (GB); Robinson, Susan, Clare, Twickenham, Middlesex TW2 6RL (GB); Dring, Neil, Charles, Medmenham, Buckinghamshire SL7 2EZ (GB); Sagel, Paul, Albert, Maineville, OH 45039 (US); Passi, Rajeev, Kumar, West Chester, OH 45069 (US); Mitra, Shekhar, Cincinnati, OH 45243 (US); Broyles, Norman Scott, Hamilton, Ohio, 45011 (US)
(74) Representative: Kohol, Sonia

(56) References cited:
- GB-A- 1 291 377
- JP-A- 9 103 320
- US-A1- 2002 146 380
- DATABASE WPI Week 198819 Thomson Scientific, London, GB; AN 1988-130170 XP002592344 & JP 63 073910 A ((NITL) NITTO ELECTRIC IND CO) 4 April 1988 (1988-04-04)
- Anonymous: "Sally Hansen, Hair remover wax strip kit for face, brows & bikini" Sally Hansen 1 August 2008 (2008-08-01), XP002600481 Retrieved from the Internet: URL:http://web.archive.org/web/20080801093 708/http://www.sallyhansen.com/product.cfm ?product=137 [retrieved on 2010-09-13]

## Description

### FIELD OF THE INVENTION

The present invention relates to cosmetic methods of removing unwanted hair from the surface of the body.

### BACKGROUND OF THE INVENTION

Depilatory compositions used to remove unwanted hair by chemical activity are known. Such compositions may comprise reducing agents to degrade keratin in the hair and thus weaken the hair strands. These compositions may take the form of creams, lotions and the like which may be applied to the unwanted hair in a variety of ways, such as with a spatula. The spatula or another suitable implement is then used to scrape off the weakened hair strands and complete the depilation process. This can be a messy and awkward procedure for the user of the depilatory cream or lotion and provides no means of occluding the depilatory composition to prevent it from drying out. By disposing the depilatory composition on a substrate one may overcome or mitigate such disadvantages. Substrate-based depilatory products are known from JP63073910A, US2006002878, JP6135826A, JP11012123A and JP62230711A. JP6192056A in particular considers water-impermeable or substantially water-impermeable substrates having some flexibility.

While addressing some of the usage problems of creams and lotions by removing the need for an application implement, known substrate-based depilatory articles do not address the problem of achieving an improved balance between ease of handling during application, and conformability to the surface of the body to which they are applied. Specifically, applicants have found that flexible substrates may collapse during handling and be difficult to accurately place on the unwanted hair, although they are able to conform to the surface of the body. Excessively rigid articles lack the capability to be conformed to the area of the body upon which there is unwanted hair, resulting in poor contact between the depilatory composition and the unwanted hair and hence an unsatisfactory hair removal process, though they may be handled with relative ease. There exists a need, therefore, for a method of depilation that is able to provide both desirable handleability and conformability.

### SUMMARY OF THE INVENTION

Surprisingly, applicants have found that a method of removing unwanted hair from the surface of the body comprising the steps of providing a depilatory article comprising a flexible substrate at least partially coated with a depilatory composition, the coating of depilatory composition forming a coated region of the depilatory article; tensioning the coated region of the depilatory article; applying the depilatory article to the surface of the body such that the coated region is applied under tension to the unwanted hair; and removing the depilatory article from the surface of the body and rubbing, scraping, rinsing, cleaning or wiping the area of the body to which the depilatory article was applied after removal of the depilatory article, wherein the flexible substrate has a mean thickness of from 50 µm to 15 µm meets the aforementioned need by being both conformable to the surface of the body and simultaneously ensuring that the depilatory article may be effectively handled during usage to ensure desired placement.

### DETAILED DESCRIPTION OF THE INVENTION

As used herein, the term "buffering base" refers to a base capable of opposing pH changes by means of chemical or physical (solubility) processes and thereby limiting the pH to less than or equal to 13 .

As used herein, the term "water impermeable" includes materials or objects through which water in its liquid state does not pass.

As used herein the term "colloid-forming" refers to chemical species that are able to form stable, aqueous solid-in-liquid colloidal systems, including nano-colloidal systems.

As used herein, the term "sodium silicate" refers to Na₂SiO₃, any other silicate comprising sodium as the only cation besides silicon, and any other silicate comprising sodium. The same definition applies correspondingly to any other silicate, for example "potassium silicate" refers to K₂SiO₃, any other silicate comprising potassium as the only cation besides silicon and any other silicate comprising potassium, "ammonium silicate" to (NH₄)₂SiO₃, any other silicate comprising ammonium as the only cation besides silicon and any other silicate comprising ammonium and "manganese silicate" to Mn₂SiO₄, any other silicate comprising manganese as the only cation besides silicon and any other silicate comprising manganese.

Methods of depilation according to the present invention comprise a step of tensioning the coated region of the depilatory article. Surprisingly, applicants have found that applying tension across the coated region of the depilatory article creates an effect of temporarily causing the coated region to appear to exhibit in increased rigidity, enabling the user to accurately position the coated region, and hence depilatory composition on to the desired region of the body. Tensioning the coated region may be achieved in a number of ways. Non-limiting examples include holding the depilatory article either side of the coated region, for example with the hands or a tool, so as to apply tension between the areas being held, holding the depilatory article at one point, for example with the hands or a tool, and allowing a weight (which may be part of the depilatory article, attached thereto or result from the weight of the depilatory composition itself) to tension the coated region. In order to apply tension by hand, the flexible substrate may comprise finger tabs positioned on substantially opposing sides of the coated region to facilitate holding of the depilatory article.

The same means used to apply tension to the coated region may be used to ensure that the depilatory article is applied to the surface of the body such that the coated region is applied under tension to the unwanted hair in order to maintain the improved handling characteristics described above. In a preferred embodiment, the tension is kept substantially constant during application of the depilatory article. The flexible nature of the substrate allows the substrate to conform to the surface of the body to offer improved contact between the depilatory composition and the unwanted hair. In a preferred embodiment, the tension may be at least partially, more preferably substantially completely released from the coated region after applying the depilatory article to the skin in order to improve the conformability of the depilatory article.

Depilatory compositions are typically less effective against longer hair than shorter hair, because of the lack of availability of depilatory actives to the hair fibres, especially near to the skin. Accordingly, the method of depilation may further comprise a step of trimming the unwanted hair before applying the depilatory article, to increase the availability of depilatory composition to the hair near the surface of the skin and prevent stubble remaining after depilation. Preferably, the hair is trimmed to a length of not more than 3 cm, more preferably not more than 2 cm, even more preferably not more than 1 cm and even more preferably still not more than 0.5 cm.

In a preferred embodiment, the method of depilation may further comprise a step of applying a pre-treatment skin care composition to the area of the body to which the depilatory article is to be applied before the depilatory article is applied and preferably immediately before the depilatory article is applied. Advantageously, the pre-treatment skin care composition may be applied after any hair trimming step. The pre-treatment skin care composition may comprise ingredients to promote skin conditioning (e.g. emollients), hair hydration or provide a skin barrier (e.g. hydrophobic materials) and intended for use prior to applying the depilatory article.

The depilatory article may be provided inside water impermeable packaging to assist in product stability prior to use, especially during transport and storage. Accordingly, the method of depilation may further comprise a step of removing the depilatory article from a water impermeable packaging.

The depilatory article may be provided with a removable release layer positioned to contact the depilatory composition on the side/face of the depilatory composition intended for application to the surface of the body. Accordingly, the method of depilation may further comprise a step of removing the removable release layer from the surface of the depilatory composition prior to application of the article to the surface of the body.

In order to improve the contact between the depilatory composition and the hair and improve depilatory efficacy, the method of depilation may further comprise a step of rubbing, dabbing, stroking or otherwise applying pressure to the surface of the depilatory article after it has been placed upon the surface of the body, preferably immediately after the depilatory article has been placed upon the surface of the body.

In order to provide sufficient time to weaken and break strands of unwanted hair but avoid irritation, the depilatory article is preferably left in contact with the skin for a period of at least 1 minute, more preferably from 2 minutes to 10 minutes, even more preferably from 3 minutes to 8 minutes.

After removing the depilatory article from the surface of the body, it may additionally be preferred to rub, wipe or scrape the surface of the body over the area to which the depilatory article, and particularly the coated region, has been applied. The rubbing, wiping or scraping may be conducted with another part of the user's body, especially the hands, or preferably with an implement such as a blunt blade or tissue. Alternatively, the rubbing, wiping or scraping may be conducted with a towel in order to dry the area of the body.

Alternatively, it may be preferred to wash the area of the body to which the depilatory article has been applied, after removal of the depilatory article. Such washing may take the form of the use of a personal cleansing composition and water, using the hands or a washing implement to provide friction across the surface of the body, or using a personal cleansing wipe. In another preferred embodiment, the surface of the body is dried (such was with a dry wipe, a tissue or a towel) after the step of cleansing the area to which the depilatory article had been applied.

The method of depilation may further comprise the step of applying a post-treatment skin care composition to the area of the body to which the depilatory article has been applied after removal of the depilatory article, after at least one of the rubbing, wiping or scraping step or the cleansing/drying step described above. The post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like as described herein above. The complementary post treatment skin care compositions may be leave-on or rinse-off compositions. The skin care compositions may also be designed to immediately follow application of the hair removal products. For example, a finishing composition may be applied to the same skin area to combat lingering odour and irritation caused by residual depilatory agent. The finishing composition may comprise a metal oxide (e.g., zinc oxide, aluminum oxide, and magnesium oxide) that is capable of complexing with any remaining depilatory agent remaining on the targeted skin area to reduce continued odour and subsequent skin irritation.

The substrate is flexible to enable conformability to the skin, and advantageously possesses a rigidity in the range of from 5.00 g/cm to 0.08 g/cm, preferably from 3.00 g/cmto 0.08 g/cm, more preferably from 1.80 g/cm to 0.10 g/cm, even more preferably from 0.80 g/cm to 0.15 g/cm and even more preferably still from 0.60 g/cm to 0.25 g/cm. This rigidity of the substrate ensures that desirable handleability and conformability attributes of a depilatory article are achieved. In particular, the article collapsing under gravity or folding is avoided, which is especially undesirable if different areas of the depilatory composition are able to readily come into contact with each other, while maintaining the capability for the substrate to conform to the surface to which it is applied without folding or crinkling, in order to further improve depilatory efficiency. Accordingly, the substrate is readily conformable to the skin and unwanted hair without permanently deforming during use, as this may also result in problems for the user during application. In a preferred embodiment, the rigidity is substantially constant and does not change during the lifetime of a product.

Rigidity can be readily measured using the American Standard Test Method (ASTM) D2923-06 on a Handle-O-Meter, model #211-300, available from Thwing-Albert Instrument Co. of Philadelphia, Pa. The rigidity is read directly from the meter and expressed as grams per centimetre of sample width. Samples were prepared as 10.16 cm (4 inch) by 10.16 cm (4 inch) test specimens with edges parallel to the machine direction and transverse direction for substrates with directionality. Three rigidity measurements were determined on the same side of fresh test specimens orientated in the same substrate direction. A further three rigidity measurements were taken on the same side of fresh test specimens oriented at 90° to the first orientation. These six measurements were repeated on the opposite side to the first six measurements, on fresh test samples. The 12 rigidity measurements were then averaged and reported to 0.01 g/cm.

The rigidity of a substrate is a function of substrate thickness and inherent modulus of elasticity. Different materials have different moduli of elasticity. Based upon the material or materials that the flexible substrate comprises, a substrate thickness should be selected that enables the desired rigidity of the flexible substrate to be achieved.

The flexible substrate may be water permeable or water impermeable. The flexible substrate may comprise any suitable material such as fibrous materials, papers, fabrics, non-wovens, plastics, amorphous solids, crystalline solids, foils, rubbers, latex, thermoplastic elastomers, cellular foams (open and closed cell), composites, laminates and mixtures thereof. Preferably, the flexible substrate is water impermeable. Using a water impermeable substrate prevents water loss from the depilatory composition while the depilatory composition is in contact with the keratinous tissue and thus prevents the depilatory composition from drying out. Water loss from the depilatory composition lowers the water concentration, thus increasing the concentration of active ingredients and bases present. This could result in irritation to the skin, which applicants wish to avoid.

The flexible substrate preferably comprises at least one water impermeable material and is compatible with depilatory compositions. Examples of useful water impermeable materials include but are not limited to polypropylene (PP); polyethylene (PE, including HDPE and LLDPE); polyethylene terephthalate (PET); polyvinylchloride (PVC); polyamide (PA); polycarbonate; polyurethane; cellulose acetate; polychloropene; polysulfone; polytetrafluoroethylene (PTFE); polyvinyl acetate (PVA); polystyrene; polyphenylene oxide (PPO); acrylonitrile butadiene styrene (ABS); acrylic; acrylonitrile styrene acrylate (ASA); ethylene vinyl alcohol (EVA); natural rubber, latex, nylon, nitrile, silicone and thermo plastic elastomers (TPE). The flexible substrate may comprise a single polymer or mixtures of polymers or copolymers. Preferably the flexible substrate comprises a plastic sheet, more preferably a polyolefin, even more preferably a polyethylene and even more preferably still high density polyethylene.

In an advantageous embodiment, the depilatory composition is disposed upon the water impermeable material, preferably plastic sheet, more preferably polyolefin, even more preferably polyethylene and even more preferably still high density polyethylene. In this advantageous embodiment, there is preferably no layer of water permeable material between the depilatory composition and the water impermeable material. In a preferred embodiment, the water impermeable material forms a water impermeable layer.

The flexible substrate preferably has a mean thickness of from 50 µm to 15 µm, more preferably from 40 µm to 16 µm and even more preferably from 30 µm to 17 µm.

Non-limiting examples of substrate material and thickness combinations for the flexible substrate are:

| **Substrate Material** | **Thickness [microns]** | **Rigidity [g/cm]** |
|---|---|---|
| HDPE | 13 | 0.13 |
| HDPE | 18 | 0.33 |
| HDPE | 36 | 1.05 |
| LLDPE | 23 | 0.23 |
| PP | 18 | 0.46 |

| | | |
|---|---|---|
| [HDPE is a mixture of LBI 85% M6030 and Exxon Mobil 15% LD2001 manufactured on a Merritt-Davis casting line] [LLDPE is Exxon Mobil 15% LD2001 manufactured on a Merritt-Davis casting line] [PP is Basell PH835 manufactured on a Merritt-Davis casting line] | | |

The flexible substrate may be a laminate comprising at least two materials, including non-wovens; paper; board; metal based substrates (eg aluminium foil); flocking or topical coatings (e.g. surfactants; printing); closed or open cell foams or substrates described herein above. In a preferred embodiment, at least one of the materials is water impermeable.

The flexible substrate may comprise a textured or, alternatively, micro-structured surface on at least a portion of one side. Surface texturing or micro-structuring increases the effective surface area of the flexible substrate and thus improves adherence of the depilatory composition to said substrate, facilitating an easy removal of the depilatory article by peeling it off the skin, or increases the grip of the surface, thus improving handleability. The textured structures may comprise dimples; lines or curvilinear embossments. A textured surface may be formed on the flexible substrate by any appropriate technique, including embossment calendars and casting.

The flexible substrate may be manufactured by any suitable method, including casting, injection moulding, co-injection moulding, over moulding, in-mold assembly, compression moulding, blow moulding, casting thermo or vacuum forming and where appropriate may be laminated by heat welding (which may further include the use of pressure, ultrasonic forces and radio or high frequencies), co-extrusion; adhesives, electro static adhesions (such as flocking by fibres) and topical surface applications.

Depilatory articles used in the present invention comprise a depilatory composition in contact with a surface of the flexible substrate, forming a coated region. The depilatory composition should be suitable for being placed in contact with a user's skin (and unwanted hair). Preferably, the depilatory composition is aqueous. The concentration of water in the depilatory composition is preferably at least 40%, more preferably from 50% to 98%, even more preferably still from 60% to 95% and yet more preferably from 70% to 90%, by weight of the depilatory composition. This high water level helps to improve the overall skin mildness of the depilatory composition by making it dilute, and by keeping the system more robust to pH changes, which may result in skin irritation.

Achieving a desired dosage of depilatory composition to the surface of the skin is a further advantage of using a substrate-based product. However, if the substrate is able to stretch or tear, the layer of depilatory composition disposed upon it may be thinned, thickened or rupture in places, resulting in uneven and hence less desirable depilatory activity. In particular, low depilatory efficacy may result in areas treated with thinned or ruptured areas of the composition while higher depilatory efficacy and increased irritation may result in areas treated with thickened areas of the composition.

The potential problem of a substrate stretching may be avoided by selecting a substrate that does not permanently deform during use. This problem may also be avoided by selecting a substrate with a sufficiently high secant modulus such that it is less likely to stretch during normal use. Accordingly, in another preferred embodiment, the substrate has a secant modulus at 2% strain of greater than 689.5 bar (10,000 psi), more preferably greater than 1379.0 bar (20,000 psi), even more preferably greater than 2068.4 bar (30,000 psi) and even more preferably still greater than 2757.9 bar (40,000 psi) in order to achieve uniform application of the depilatory composition to the surface of the body during usage. Without wishing to be bound by theory, applicants believe that using a substrate with an excessively low secant modulus at 2% strain can deform and thus break apart the depilatory composition disposed on the substrate, leading to uneven depilatory action and increased risk of irritation. The secant modulus at 2% strain may be measured readily using the American Standard Test Method (ASTM)) 'Standard Test Method for Tensile Properties of Thin Plastic Sheeting D882-09' conducted on an MTS Insight1 Tensile Tester available from MTS Systems Co, Eden Prairie, MN, USA. This method may also be applied to non-plastic materials and is designed for use on sheets with a thickness of less than 1 mm.

The potential problem of a substrate tearing may be avoided by selecting a substrate that does not fail during usage. This problem may also be avoided by selecting a substrate with a sufficiently high nominal tensile strength such that it is less likely to tear during normal use. Accordingly, in another preferred embodiment, the substrate has a nominal tensile strength of at least 5 MPa more preferably at least 10 MPa even more preferably at least 15 MPa and even more preferably still at least 18 MPa in order to achieve uniform application of the depilatory composition to the surface of the body during usage. Without wishing to be bound by theory, applicants believe that using a substrate with an excessively low nominal tensile strength can fail during usage and thus break apart the depilatory composition disposed on the substrate, leading to uneven depilatory action and increased risk of irritation. The nominal tensile strength may be measured readily using the American Standard Test Method (ASTM) 'Standard Test Method for Tensile Properties of Thin Plastic Sheeting D882-09' conducted on an MTS Insight Tensile Tester available from MTS Systems Co, Eden Prairie, MN, USA. This method may also be applied to non-plastic materials and is designed for use on sheets with a thickness of less than 1 mm.

Preferably, the depilatory composition is disposed upon the flexible substrate in an amount per unit area of 0.300 g/cm² to 0.001 g/cm², more preferably from 0.015 g/cm² to 0.003 g/cm², even more preferably from 0.080 g/cm² to 0.005 g/cm² and even more preferably still from 0.05 g/cm² to 0.005 g/cm², wherein the unit area refers to the coated region of the flexible substrate and not including any uncoated surface of the flexible substrate. Additionally, the area used to calculate the amount of depilatory composition disposed upon the flexible substrate is calculated ignoring any surface texturing or micro-structuring. Alternatively, the mean thickness of the depilatory composition is preferably from 0.01 mm to 3 mm, more preferably 0.1 mm to 1.5 mm, even more preferably from 0.05 mm to 0.8 mm, and even more preferably still from 0.05 mm to 0.5 mm.

A layer of depilatory composition can be applied to the flexible substrate through any known technique of applying viscous fluids to substrates, including, for example, extrusion, casting (e.g., reverse roll, knife-over roll, slot die, Gravure roll), spraying, knife blade coating, and zone coating. Such techniques may be modified to alter the quantity of depilatory composition disposed on the flexible substrate. For example, the speed at which the flexible substrate travels through an extrusion process determines the quantity of depilatory composition disposed upon said substrate. The area of depilatory composition may cover the entire surface of the flexible substrate of a portion thereof. Advantageously, the depilatory composition covers less than the entire surface of the flexible substrate to facilitate handling. The flexible substrate may comprise at least one region with two orthogonal dimensions each of a length greater than 1 cm, preferably greater than 1.5 cm and more preferably greater than 2 cm upon which no depilatory composition is disposed.

In a preferred embodiment, the depilatory composition comprises a keratin reducing agent to weaken and/or break strands of unwanted hair. Non-limiting examples of suitable keratin reducing agents include: sulphide salts such as Li₂S, Na₂S, K₂S, MgS, CaS, SrS or BaS, hydrogen sulphide salts such as NaSH or KSH, thioglycol, thioglycerol, thioglycolamide, thioglycolhydrazide, thioglycolic acid, thioglycolate salts (such as potassium thioglycolate, calcium thioglycolate, ammonium thioglycolate, diammonium dithioglycolate, glyceryl monothioglycolate, or monoethanolamine thioglycolate), thiosalicylic acid, thiomalic acid, ammonium thiolactate, monoethanolamine thiolactate, dithioerythritol, 2-mercaptopropionic acid, 1,3-dithiopropanol, glutathione, dithiothreitol, cysteine, homocysteine, N-acetyl-L-cysteine and cysteamine. Advantageously, the keratin reducing agent is present in an amount of from 0.3% to 20%, preferably from 0.8% to 15%, more preferably from 1% to 10% by weight of the composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof In a preferred embodiment, the concentration of the conjugate acid of the thioglycolate salt is from 0.5% to 12.0%, more preferably from 0.8% to 8.0% and even more preferably from 1.0% to 6.0% by weight of the depilatory composition.

In a preferred embodiment, the depilatory composition comprises a monovalent cation, preferably a monovalent metal cation. Without wishing to be bound by theory, the applicants believe that the presence of monovalent metal cations increases the dissociation of thioglycolate salts. The monovalent cations such as those derived from monovalent cation containing salts are able to displace the cation of the thioglycolate salt and further enhance dissociation of said thioglycolate salt. This increases the amount of deprotonated thioglycolate formed from the thioglycolate salt and therefore increases the effectiveness of the depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

In order to further enhance the safety of the resulting product, it is advantageous to limit the amount of monovalent cations, preferably monovalent metal cations, to which the skin is exposed when the depilatory article is used, although a small quantity may improve the efficacy of the depilatory composition. Advantageously, the quantity of monovalent cations (or monovalent metal cations in the preferred embodiment above) per unit area of the aforementioned coated region is less than 5.10 x 10⁻⁴ mol/cm², preferably less than 3x10⁻⁴ mol/cm⁻², more preferably from 1 x 10⁻⁹ mol/cm² to 1.5 x 10⁻⁴ mol/cm², even more preferably from 2.50 x 10⁻⁸ mol/cm² to 6.65 x 10⁻⁵ mol/cm² and even more preferably still from 6 x 10⁻⁷ moVcm2 to 4.5 x 10⁻⁵ mol/cm². The selection of keratin reducing agent and optional ingredients including the base may be made considering the quantity of monovalent cations or monovalent metal cations achieved.

Limiting the quantity of monovalent ion present in the depilatory composition may prevent skin irritation but also limits the quantity of thioglycolate salt that may be present in a formula if monovalent ion containing thioglycolate salts or bases are used. Accordingly, in an advantageous embodiment, the depilatory composition comprises a divalent cation,, preferably a divalent metal cation, and preferably wherein the thioglycolate salt, the buffering base (if present) or both comprises a divalent cation, or more preferably a divalent metal cation in order to enable the inclusion of additional depilatory active. In another preferred embodiment, the thioglycolate salt comprises a divalent metal cation. Applicants have established that thioglycolate salts comprising monovalent metal cations, such as potassium thioglycolate, are effective at removing hair from the skin, even at low doses, but may expose the skin tissue to harsh chemical conditions, resulting in irritation. On the other hand, thioglycolate salts comprising divalent metal cations, such as calcium thioglycolate, are relatively non-irritating to the skin.

In a depilatory composition comprising a mixture of monovalent and divalent ions, controlling the ratio of divalent ions to monovalent ions may also improve the safety characteristics of the depilatory articles used in the present invention. Increasing the concentration of divalent ions relative to the concentration of monovalent ions increases the likelihood that any particular depilatory active species is associated with a divalent ion, rather than the more irritating monovalent ions. On the other hand, increasing the concentration of monovalent ions increases the effectiveness of the depilatory composition. Accordingly, in an alternative embodiment the ratio of the concentration of divalent ions to the concentration of monovalent ions present in the depilatory composition is advantageously in the range of from 400:1 to 0.02:1, preferably from 200:1 to 0.1:1, more preferably 60:1 to 0.3:1, even more preferably from 20:1 to 0.5:1, and even more preferably still from 15:1 to 1:1.

The pH of the depilatory composition may advantageously be in the range of from 5 to 13.8, preferably from greater than 7 to 13, more preferably from 9 to 12.9, even more preferably from 10 to 12.8, even more preferably still from 12 to 12.7 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof Even more preferably still, the depilatory composition comprises calcium hydroxide.

In a preferred embodiment, the base is present at a concentration of from 0.1% to 10.0%, more preferably from 0.5% to 8.0% and even more preferably from 1.0% to 5.0%, by weight of the depilatory composition.

In another preferred embodiment, the depilatory composition comprises at least one silicate or silica, advantageously at least one water-soluble or colloid-forming silicate or silica.

Preferably, the depilatory composition comprises at least one water-soluble or colloid-forming silicate selected from lithium silicates; sodium silicates (including disodium metasilicate pentahydrate and disodium metasilicate nanohydrate); potassium silicates; calcium silicates, ammonium silicates; manganese silicates; imidazolium silicates, synthetic and natural silicates (clays) or mixtures thereof. More preferably, the depilatory composition comprises at least one water-soluble or colloid-forming silicate selected from synthetic clays; sodium silicates, potassium silicates, or mixtures thereof and even more preferably the depilatory composition comprises a sodium silicate or mixtures of sodium silicates.

Alternatively, the depilatory composition comprises a form of silica that is colloid-forming, (such as amorphous microporous silica), forms sol or gel systems, (such as silica gels and nano-colloidal silicas), or is mesostructured. Surface modification of silica may be advantageous to promote the formation of stable colloid systems.

Suitable synthetic and natural silicates (clays) are available commercially as: Laponite® RDS; XLS and S etc. (available from RockWood Additives Limited); Wyoming Bentonite; Californian Hectorite; Jadeite; Enstaite and Rhodonite; Benonate® EW (available from Rheox Inc.); Bentolite® (available from Southern Clay Products Inc.) Optigel® (available from Süd Chemie Rheologicals)

The silicate or silica is preferably present in the depilatory composition in an amount per unit area of the coated region of from 2.05 x 10⁻⁸ mol/cm² to 1.23 x 10⁻⁴ mol/cm², preferably from 1.64 x 10⁻⁷ mol/cm² to 3.69 x 10⁻⁵ mol/cm² and more preferably from 4.92 x 10⁻⁷ mol/cm² to 8.20 x 10⁻⁶ mol/cm². Within the preferred ranges, the effectiveness of the depilatory composition is further increased while irritation is maintained within an acceptable level. Without wishing to be bound by theory, applicants believe that an amount of silicate or silica is required in order to enhance the dissociation of the thioglycolate salt sufficiently for the increase in efficacy to be clearly apparent to the user, but that excessive dosage of silicate or silica may lead to over-dissociation of the thioglycolate salt resulting in increased skin irritation. Alternatively, the silicate or silica may be present in the depilatory composition in an amount of from 0.01% to 5%, preferably 0.1% to 4%, more preferably 0.2% to 3% and even more preferably from 0.5% to 2% by weight of the depilatory composition.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the depilatory composition. It may be desirable to utilize gel network structures or oil-in-water emulsions to thicken the depilatory compositions. Suitable materials for preparing the gel network structures or oil-in-water emulsions are well represented in the art and include fatty materials such as fatty alcohols (for example cetyl alcohol and stearyl alcohol) alone or used in conjunction with non-polar oils such as paraffin or mineral oils. An appropriate emulsifier may also be used to form and stabilize the bilayer structure characteristic of gel network structures or to form and stabilize an oil-in-water emulsion. The thickening agent may be present at a level of from about 0.01 % to about 20%, preferably from about 0.1 % to about 10%, more preferably from about 0.3% to about 5%, and even more preferably from about 0.5% to about 4%, by weight of the aqueous depilatory composition.

Advantageously, the thickening agent comprises carrageenan. The carrageenan is preferably present in an amount of from 0.1% to 10%, more preferably from 0.5% to 8%, even more preferably from 1% to 5% and even more preferably still from 2% to 4% by weight of the depilatory composition. The carrageenan may be iota, kappa or lambda carrageenan, and in a preferred embodiment is iota carrageenan. Without wishing to be bound by theory, the applicants believe that a depilatory composition comprising carrageenan has both an affinity to the surface of the skin, providing an effect analogous to a frictional resistance opposing spreading of the composition and cohesive forces that further prevent spreading and additionally prevent rupturing of the composition.

The rheological properties of the depilatory composition may also lead to improved performance in use. In particular, the yield point describes the resistance of the depilatory composition to deformation under environmental stress. If the yield point is too high, then the depilatory composition may not deform sufficiently, with hair fibres unable to enter the depilatory composition effectively upon application, resulting in less desirable depilatory effectiveness. If the yield point is too low, however, then the depilatory composition may flow during storage, transport or use and is not cleanly removed from the skin upon removal of the depilatory article, thus requiring the inconvenience of additional wiping and risking irritation to the user. Accordingly, the phase angle of the depilatory composition preferably has a yield point from 10 Pa to 2000 Pa, more preferably from 30 Pa to 1200 Pa, even more preferably from 45 Pa to 500 Pa and even more preferably still from 60 Pa to 300 Pa, when measured via a stress controlled amplitude sweep at a frequency of 1 Hz and a temperature of 25°C. The yield point described is defined as the 5% decrease in magnitude of the elastic modulus G' linear viscoelastic plateau value as measured on a TA1000 Rheometer, available from TA Instruments of New Castle, Delaware, USA. The rheological properties of the depilatory composition may be altered by changing the concentration or identity of the thickening system and the water content of the depilatory composition.

Advantageously, the depilatory composition displays an elastic modulus G' which exceeds its viscous modulus G" at all frequencies below 60 rad/s, preferably below 20 rad/s, more preferably below 10 rad/s and even more preferably below 1 rad/s; when measured via a strain controlled frequency sweep; at a strain of 1% and a temperature of 25°C. The elastic modulus of the depilatory composition exceeds its viscous modulus at a low frequency of applied stress. This indicates that the depilatory composition is behaving in a solid-like manner at rest and is of particular benefit when the depilatory composition is interposed between two substrates, for example a substrate and a protective release layer.

In another preferred embodiment, the depilatory composition displays a high degree of shear thinning behaviour enabling the effective coating of target hairs during application and improve depilatory efficacy. Accordingly, at a low shear rate of 0.1 s⁻¹, the dynamic viscosity of the depilatory composition is preferably 1000 Pa.s to 10000 Pa.s measured at a temperature of 25°C, whereas at a high shear rate of 1000 s⁻¹, the dynamic viscosity of the depilatory composition is preferably 0.1 Pa.s to 1 Pa.s, measured at a temperature of 25°C.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter and glycerine); skin rejuvenation compositions (for example targeted for fine lines, wrinkles and uneven skin tone including retinoids), cosmetic compositions; anti-inflammatory agents (including corticosteroids); topical anaesthetics (for example benzocaine and lidocaine); antioxidants (including flavonoids) radical scavengers; sunscreen agents; skin cooling or warming agents and the like. The depilatory composition may comprise one or more skin care ingredients present in an amount of from about 0.001 % to about 10%, more preferably from about 0.01 % to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in hair removal compositions particularly dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

Depilatory articles used in the present invention may take any form suitable for applying to keratinous tissue. The size and shape of the depilatory article may take any form suitable for application to the body area from which hair is to be removed. The depilatory article will preferably relate to the body area or zone from which hair is to be removed, especially the face (including the jaw, chin and upper lip regions of the face), underarm and bikini areas. Preferably, the depilatory article takes the form of a mask (configured for the face) or a strip/patch (configured for general use). In another preferred embodiment, the substrate of the depilatory article is substantially planar.

The coated region preferably comprises an upper-lip portion adapted to be placed above a human mouth, and a first return portion projecting from the upper lip portion and adapted to be placed contiguously with the outer extremity of the vermilion lip in a first corner of the mouth. The return portion has a length along its greatest dimension of at least 0.2 cm, preferably from 0.5 cm to 5 cm, more preferably from 0.75 cm to 4 cm, even more preferably from 1 cm to 3 cm. Applicants have found that this configuration enables the user to remove unwanted hair from the skin immediately surrounding the corner of the mouth while lowering the risk of depilatory composition contacting the vermillion lip, where it may cause irritation. In an alternative embodiment, the coated region further comprises a second return portion projecting from the upper lip portion and adapted to be placed contiguously with the outer extremity of the vermillion lip in a second corner of the mouth.

Advantageously, the upper lip portion has a length along its greatest dimension of at least 0.2 cm, preferably from 0.5 cm to 15 cm, more preferably from 1 cm to 12 cm, even more preferably from 2 cm to 10 cm and even more preferably still from 3 cm to 8 cm. This dimension enables the upper lip portion to cover a desirable length of the upper lip and thus achieve the desired depilatory action. In a preferred embodiment, the upper lip portion is adapted to be placed to be at least partially contiguously with the upper border of the upper vermilion lip, to enable depilatory action to be achieved on the skin immediately surrounding the upper vermilion lip while lowering the risk of depilatory composition contacting the upper vermilion lip, where it may cause irritation.

In another preferred embodiment, the coated region comprises a lower lip portion adapted to be placed below a human mouth, preferably wherein the lower lip portion is adapted to be placed to be least partially contiguously with the lower border of the lower vermilion lip to enable depilatory action to be achieved on the skin immediately surrounding the lower vermilion lip while lowering the risk of depilatory composition contacting the lower vermilion lip, where it may cause irritation.

Depilatory articles used in the present invention may comprise at least two finger-tabs being substantially free of depilatory composition and positioned on substantially opposing sides of the coated region. These finger tabs enable a user to apply tension to the coated region of the flexible substrate. Surprisingly, applicants have found that applying tension across the coated region of the depilatory article creates an effect of temporarily causing the coated region to exhibit an apparent increased rigidity, enabling the user to accurately position the coated region, and hence depilatory composition on to the desired region of the body. Tensioning the coated region may be achieved in a number of ways, non-limiting examples of which include holding the depilatory article either side of the coated region, for example with the hands or a tool, so as to apply tension between the areas being held. Alternatively, depilatory articles used in the present invention may comprise at least one finger-tab being substantially free of depilatory composition and positioned to allow the weight of the article to tension the coated region when being held by the finger-tab.

In a preferred embodiment, at least one finger tab extends from the nearest perimeter of the coated region by a minimum of 1 cm, preferably from 1.5 cm to 5 cm, more preferably from 2 cm to 4 cm and even more preferably from 2.5 cm to 3.5 cm. In another preferred embodiment, both finger-tabs extend from the nearest perimeter of the coated region by a minimum of 1 cm, preferably from 1.5 cm to 5 cm, more preferably from 2 cm to 4 cm and even more preferably from 2.5 cm to 3.5 cm, in order to aid handling of the depilatory article.

Depilatory articles used in the present invention may comprise a protective release layer removably attached to the depilatory composition, preferably on a surface of the depilatory composition substantially opposing that which is in contact with the flexible substrate. The protective release layer may comprise materials including polymer resins such as a polyolefins e.g. polypropylene (including stratified biaxially oriented polypropylene (SBOPP)), polyethylene (including LDPE; LLDPE; HDPE; Metallocene) or polyethylene terephthalate. Alternative materials which may be used include polyvinylchloride, polyamide, acetyl, acrylonitrile butadiene styrene, acrylic, acrylonitrile styrene acrylate, ethylene vinyl alcohol, ethylene vinyl acetate, Nylon, Latex, natural or synthetic rubbers, polycarbonate, polystyrene, silicone or thermo plastic elastomer, thermo plastic vulcanate or copolymers of said materials. Where appropriate the protective release layer may comprise one or more laminations, combinations of multiple layers and/or indications (which may include instructions and illustrations) relating to at least one aspect of the usage of the depilatory article. In an advantageous the protective release layer may comprise a coating of a non-stick material. Exemplary non-stick coatings include wax, silicone, fluoropolymers such as TEFLON®, and fluorosilicones. In a preferred embodiment, the protective release layer covers at least the entire aforementioned coated region of the flexible substrate. In another preferred embodiment the protective release layer is water impermeable. In a further preferred embodiment, the protective release layer has a mean thickness of at least 85 microns, more preferably from 85 microns to 130 microns, even more preferably from 90 microns to 120 microns. In yet another preferred embodiment, the protective release layer extends beyond the coated region of the flexible substrate to provide a removal tab.

In a preferred embodiment, the depilatory articles used in the present invention are packaged to prevent water loss and/or oxygen permeation. Alternatively, the depilatory articles used in the present invention are packaged in water impermeable packaging. Examples of suitable packaging materials include films of EVOH; PP; PE; Nylon; foil laminates (including metalized PET; BOPP and PE), mixtures thereof, laminates thereof or multi-laminates thereof More preferably, the packaging comprises an inert gas and even more preferably the inert gas comprises at least one of nitrogen, argon or carbon dioxide. Alternatively, the packaging comprises a partial vacuum.

### Example

The following example further describes and demonstrates an article usable in one embodiment within the scope of the present invention. The example is given solely for the purpose of illustration and is not to be construed as a limitation of the present invention, as many variations thereof are possible.

| Formulation Ingredients | % w/w |
|---|---|
| DI water | 84.42 |
| Acrylic Acid / VP Crosspolymer (Ultrathix P-100)¹ | 3.00 |
| Sodium Silicate (42% w/w in water) (Cognis 60)² | 2.08 |
| Calcium Hydroxide³ | 4.50 |
| Calcium Thioglycolate Trihydrate⁴ | 6.00 |

| | |
|---|---|
| 1 Ultrathix P-100 available from International Specialty Products Inc. (ISP) 2 Sodium Silicate (Cognis 60) available from Cognis 3 Calcium Hydroxide Reag. Ph. Eur. puriss. p.a. available from Sigma-Aldrich Co. 4 Calcium Thioglycolate Trihydrate 99.8% available from BRUNO BOCK Chemische Fabrik GmbH & Co. | |

A 400 ml speed mixer plastic pot was sanitized and DI water weighed in directly. The Calcium Hydroxide was added with mixing and the batch was then heated to 37 °C in a water bath for 10 minutes. The Ultrathix P-100 was then slowly added to the batch in portions over 7 min (increasing the mixing speed if required). The batch was mixed for a further 10 min (again, increasing the missing speed as required). The batch was then cooled to room temperature using a water jacket and the Sodium Silicate was added slowly followed by the Calcium Thioglycolate. After mixing for a further 10 min to ensure full incorporation of the Calcium Thioglycolate and batch homogeneity, the batch was transferred to a thick walled 400 ml glass beaker and milled for 2 minutes using an IKA T50 (5,200 rpm).

The above formulation was disposed to a thickness of 0.3 mm, width of 3.0 cm and length of 3.5 cm on a cast HDPE 85% LLDPE 15% polymer blend film (manufactured on a Merritt-Davis casting line with LBI 85% M6030 and Exxon Mobil 15% LD2001; 23 microns in thickness and cut to be 9.5 cm in length and 3.2 cm in width) using a stencil and wiper blade, such that the area covered by the inventive formulation was centered along both the length and width of the film such as to afford a 3 cm finger tab at either end of the length of the coating of formulation. The cast HDPE 85% LLDPE 15% polymer blend film has a rigidity of 0.47 g/cm as measured on a Handle-o-Meter according to the American Standard Test Method (ASTM) D2923-06.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method of removing unwanted hair from the surface of the body comprising the steps of:
a) providing a depilatory article comprising a flexible substrate at least partially coated with a depilatory composition, the coating of depilatory composition forming a coated region of the depilatory article;
b) tensioning the coated region of the depilatory article, preferably by hand;
c) applying the depilatory article to the surface of the body such that the coated region is applied under tension to the unwanted hair; and
d) removing the depilatory article from the surface of the body
e) rubbing, scraping, rinsing, cleaning or wiping the area of the body to which the depilatory article was applied after removal of the depilatory article
wherein the flexible substrate has a mean thickness of from 50 µm to 15 µm.

2. A method of removing unwanted hair from the surface of the body according to claim 1, further comprising the step of rubbing, dabbing, stroking or otherwise applying pressure to the surface of the depilatory article after it has been placed upon the surface of the body, preferably immediately after the depilatory article has been placed upon the surface of the body.

3. A method of removing unwanted hair from the surface of the body according to claim 1, further comprising the step of leaving the depilatory article applied to the surface of the body for at least 1 minute, preferably for from 2 minutes to 10 minutes and more preferably from 3 minutes to 8 minutes.

4. A method of removing unwanted hair from the surface of the body according to either preceding claim, further comprising the step of at least partially releasing the tension from the coated region of the depilatory article after applying the depilatory article to the surface of the body.

5. A method of removing unwanted hair from the surface of the body according to any preceding claim, further comprising the step of removing a removable release layer from the surface of the depilatory composition prior to applying the depilatory article to the surface of the body.

6. A method of removing unwanted hair from the surface of the body according to any preceding claim, further comprising the step of trimming the hair to be removed to a length of not more than 3 cm, preferably not more then 2 cm, more preferably not more than 1 cm and even more preferably not more than 0.5 cm, prior to the step of tensioning the coated region of the depilatory article.

7. A method of removing unwanted hair from the surface of the body according to any preceding claim, further comprising the step of applying a pre-treatment skin care composition to the area of the body to which the depilatory article is to be applied prior to the step of tensioning the coated region of the depilatory article.

8. A method of removing unwanted hair from the surface of the body according to any preceding claim, further comprising the step of applying a post-treatment skin care composition to the area of the body to which the depilatory article was applied after the step of removing the depilatory article from the surface of the body.

9. A method of removing unwanted hair from the surface of the body according to any preceding claim, further comprising the step of rubbing, scraping, rinsing, cleaning or wiping the area of the body to which the depilatory article was applied after removal of the depilatory article before the application of a post-treatment skin care composition to the area of the body to which the depilatory article was applied.

10. A method of removing unwanted hair from the surface of the body according to any preceding claim, wherein the flexible substrate has a rigidity in the range of from 5.00 g/cm to 0.08 g/cm, preferably from 3.00 g/cm to 0.08 g/cm, more preferably from 1.80 g/cm to 0.10 g/cm, even more preferably from 0.80 g/cm to 0.15 g/cm and even more preferably still from 0.60 g/cm to 0.25 g/cm.

11. A method of removing unwanted hair from the surface of the body according to any preceding claim, wherein the flexible substrate has a secant modulus at 2% strain of greater than 689.5 bar (10,000 psi), more preferably greater than 1379.0 bar (20,000 psi), even more preferably greater than 2068.4 bar (30,000 psi) and even more preferably still greater than 2757.9 bar (40,000 psi).

12. A method of removing unwanted hair from the surface of the body according to any preceding claim, wherein the flexible substrate has a nominal tensile strength of at least 5 MPa, preferably at least 10 MPa, more preferably at least 15 MPa and even more preferably at least 18 MPa.

13. A method of removing unwanted hair from the surface of the body according to any preceding claim, wherein the depilatory article comprises at least two finger tabs positioned on substantially opposing sides of the coated region.

14. A method of removing unwanted hair from the surface of the body according to any preceding claim, wherein the flexible substrate is water impermeable, preferably comprises a plastic sheet, more preferably a polyolefin, even more preferably a polyethylene and even more preferably still high density polyethylene.

15. A method of removing unwanted hair from the surface of the body according to any preceding claim, wherein the depilatory composition is disposed on the flexible substrate in an amount per unit area of the coated region of 0.300 g/cm² to 0.001 g/cm², more preferably from 0.015 g/cm² to 0.003 g/cm², even more preferably from 0.080 g/cm² to 0.005 g/cm² and even more preferably still from 0.05 g/cm² to 0.005 g/cm².

## Patentansprüche

1. Verfahren zum Entfernen unerwünschter Haare von der Körperoberfläche, das folgende Schritte umfasst:
a) Bereitstellen eines Depilierartikels, der ein elastisches Substrat umfasst, das zumindest teilweise mit einer Depilierzusammensetzung beschichtet ist, wobei die Beschichtung mit der Depilierzusammensetzung einen beschichteten Bereich des Depilierartikels bildet;
b) Spannen des beschichteten Bereichs des Depilierartikels, vorzugsweise per Hand;
c) Aufbringen des Depilierartikels auf die Hautoberfläche solcherart, dass der beschichtete Bereich unter Spannung auf die unerwünschten Haare aufgebracht wird; und
d) Entfernen des Depilierartikels von der Körperoberfläche
e) Reiben, Schaben, Abspülen, Reinigen oder Abwischen des Körperbereichs, auf den der Depilierartikel aufgebracht wurde, unter Entfernen des Depilierartikels,
wobei das elastische Substrat eine mittlere Stärke von 50 µm bis 15 µm aufweist.

2. Verfahren zum Entfernen unerwünschter Haare von der Körperoberfläche nach Anspruch 1, das ferner den Schritt eines Reibens, Tupfens, Streichens oder anderweitigen Aufbringens von Druck auf die Oberfläche des Depilierartikels nach dessen Aufbringen auf die Körperoberfläche umfasst, vorzugsweise direkt nachdem der Depilierartikel auf die Körperoberfläche aufgebracht wurde.

3. Verfahren zum Entfernen unerwünschter Haare von der Körperoberfläche nach Anspruch 1, das ferner den Schritt eines Belassens des auf die Körperoberfläche aufgebrachten Depilierartikels über einen Zeitraum von mindestens 1 Minute, vorzugsweise 2 Minuten bis 10 Minuten und mehr bevorzugt 3 Minuten bis 8 Minuten umfasst.

4. Verfahren zum Entfernen unerwünschter Haare von der Körperoberfläche nach einem der vorstehenden Ansprüche, das ferner den Schritt eines zumindest teilweisen Lösens der Spannung in dem beschichteten Bereich des Depilierartikels nach dem Aufbringen des Depilierartikels auf die Körperoberfläche umfasst.

5. Verfahren zum Entfernen unerwünschter Haare von der Körperoberfläche nach einem der vorstehenden Ansprüche, das ferner den Schritt eines Entfernens einer lösbaren Abziehschicht von der Oberfläche der Depilierzusammensetzung vor dem Aufbringen des Depilierartikels auf die Körperoberfläche umfasst.

6. Verfahren zum Entfernen unerwünschter Haare von der Körperoberfläche nach einem der vorstehenden Ansprüche, das ferner den Schritt eines Stutzen der zu entfernenden Haare auf eine Länge von höchstens 3 cm, vorzugsweise höchstens 2 cm, mehr bevorzugt höchstens 1 cm und noch mehr bevorzugt höchstens 0,5 cm vor dem Schritt des Spannens des beschichteten Bereichs des Depilierartikels umfasst.

7. Verfahren zum Entfernen unerwünschter Haare von der Körperoberfläche nach einem der vorstehenden Ansprüche, das ferner den Schritt eines Aufbringens einer Hautvorbehandlungszusammensetzung auf den Körperbereich, auf den der Depilierartikel aufgebracht werden soll, vor dem Schritt eines Spannens des beschichteten Bereichs des Depilierartikels umfasst.

8. Verfahren zum Entfernen unerwünschter Haare von der Körperoberfläche nach einem der vorstehenden Ansprüche, das ferner den Schritt eines Aufbringens einer Hautnachbehandlungszusammensetzung auf den Körperbereich, auf den der Depilierartikel aufgebracht wurde, nach dem Schritt eines Entfernens des Depilierartikels von der Körperoberfläche umfasst.

9. Verfahren zum Entfernen unerwünschter Haare von der Körperoberfläche nach einem der vorstehenden Ansprüche, das ferner den Schritt eines Reibens, Schabens, Abspülens, Reinigens oder Abwischens des Körperbereichs, auf den der Depilierartikel aufgebracht wurde, nach Entfernen des Depilierartikels vor dem Aufbringen einer Hautnachbehandlungszusammensetzung auf den Körperbereich, auf den der Depilierartikel aufgebracht wurde, umfasst.

10. Verfahren zum Entfernen unerwünschter Haare von der Körperoberfläche nach einem der vorstehenden Ansprüche, wobei das elastische Trägermaterial eine Steifigkeit in einem Bereich von 5,00 g/cm bis 0,08 g/cm, vorzugsweise von 3,00 g/cm bis 0,08 g/cm, mehr bevorzugt von 1,80 g/cm bis 0,10 g/cm, noch mehr bevorzugt von 0,80 g/cm bis 0,15 g/cm und sogar noch mehr bevorzugt von 0,60 g/cm bis 0,25 g/cm aufweist.

11. Verfahren zum Entfernen unerwünschter Haare von der Körperoberfläche nach einem der vorstehenden Ansprüche, wobei das elastische Substrat einen Sekansmodul bei 2 % Belastung von über 68,9 MPa (689,5 bar (10.000 psi)), mehr bevorzugt mehr als 137,9 MPa (1379,0 bar (20.000 psi)), noch mehr bevorzugt mehr als 206,8 MPa (2068,4 bar (30.000 psi)) und sogar noch mehr bevorzugt mehr als 275,8 MPa (2757,9 bar (40.000 psi)) aufweist.

12. Verfahren zum Entfernen unerwünschter Haare von der Körperoberfläche nach einem der vorstehenden Ansprüche, wobei das elastische Substrat eine Nennzugfestigkeit von mindestens 5 MPa, vorzugsweise mindestens 10 MPa, mehr bevorzugt mindestens 15 MPa und noch mehr bevorzugt mindestens 18 MPa aufweist.

13. Verfahren zum Entfernen unerwünschter Haare von der Körperoberfläche nach einem der vorstehenden Ansprüche, wobei der Depilierartikel mindestens zwei Fingerlaschen an im Wesentlichen einander entgegengesetzt liegenden Seiten des beschichteten Bereichs umfasst.

14. Verfahren zum Entfernen unerwünschter Haare von der Körperoberfläche nach einem der vorstehenden Ansprüche, wobei das elastische Substrat wasserundurchlässig ist und vorzugsweise eine Plastikfolie umfasst, mehr bevorzugt ein Polyolefin, noch mehr bevorzugt ein Polyethylen und sogar noch mehr bevorzugt Niederdruckpolyethylen.

15. Verfahren zum Entfernen unerwünschter Haare von der Körperoberfläche nach einem der vorstehenden Ansprüche, wobei die Depilierzusammensetzung auf dem elastischen Substrat in einer Menge pro Einheitsfläche am beschichteten Bereich von 0,300 g/cm² bis 0,001 g/cm², mehr bevorzugt von 0,015 g/cm² bis 0,003 g/cm², noch mehr bevorzugt von 0,080 g/cm² bis 0,005 g/cm² und sogar noch mehr bevorzugt von 0,05 g/cm² bis 0,005 g/cm² aufgebracht wird.

## Revendications

1. Procédé d'élimination des poils indésirables de la surface du corps, comprenant les étapes consistant à :
a) fournir un article dépilatoire comprenant un substrat souple au moins partiellement revêtu d'une composition dépilatoire, le revêtement de composition dépilatoire formant une région revêtue de l'article dépilatoire ;
b) tendre la région revêtue de l'article dépilatoire, de préférence à la main ;
c) appliquer l'article dépilatoire sur la surface du corps de telle sorte que la région revêtue est appliquée sous tension sur les poils indésirables ; et
d) retirer l'article dépilatoire de la surface du corps
e) frotter, gratter, rincer, nettoyer ou essuyer la zone du corps sur laquelle l'article dépilatoire a été appliqué après retrait de l'article dépilatoire
dans lequel le substrat souple a une épaisseur moyenne allant de 50 µm à 15 µm.

2. Procédé d'élimination des poils indésirables de la surface du corps selon la revendication 1, comprenant, en outre, l'étape consistant à frotter, tamponner, masser ou autrement appliquer une pression sur la surface de l'article dépilatoire après qu'il a été placé sur la surface du corps, de préférence immédiatement après que l'article dépilatoire a été placé sur la surface du corps.

3. Procédé d'élimination des poils indésirables de la surface du corps selon la revendication 1, comprenant, en outre, l'étape consistant à laisser l'article dépilatoire appliqué sur la surface du corps pendant au moins 1 minute, de préférence de 2 minutes à 10 minutes et plus préférablement de 3 minutes à 8 minutes.

4. Procédé d'élimination des poils indésirables de la surface du corps selon l'une ou l'autre revendication précédente, comprenant, en outre, l'étape consistant à libérer au moins partiellement la tension de la région revêtue de l'article dépilatoire après application de l'article dépilatoire sur la surface du corps.

5. Procédé d'élimination des poils indésirables de la surface du corps selon l'une quelconque des revendications précédentes, comprenant, en outre, l'étape consistant à retirer une couche détachable amovible de la surface de la composition dépilatoire avant d'appliquer l'article dépilatoire sur la surface du corps.

6. Procédé d'élimination des poils indésirables de la surface du corps selon l'une quelconque des revendications précédentes, comprenant, en outre, l'étape consistant à couper les poils à enlever à une longueur qui n'est pas plus de 3 cm, de préférence pas plus de 2 cm, plus préférablement pas plus de 1 cm et encore plus préférablement pas plus de 0,5 cm, avant l'étape consistant à tendre la région revêtue de l'article dépilatoire.

7. Procédé d'élimination des poils indésirables de la surface du corps selon l'une quelconque des revendications précédentes, comprenant, en outre, l'étape consistant à appliquer une composition de soin de la peau de prétraitement sur la zone du corps sur laquelle l'article dépilatoire doit être appliqué avant l'étape consistant à tendre la région revêtue de l'article dépilatoire.

8. Procédé d'élimination des poils indésirables de la surface du corps selon l'une quelconque des revendications précédentes, comprenant, en outre, l'étape consistant à appliquer une composition de soin de la peau de post-traitement sur la zone du corps sur laquelle l'article dépilatoire a été appliqué après l'étape consistant à retirer l'article dépilatoire de la surface du corps.

9. Procédé d'élimination des poils indésirables de la surface du corps selon l'une quelconque des revendications précédentes, comprenant, en outre, l'étape consistant à frotter, gratter, rincer, nettoyer ou essuyer la zone du corps sur laquelle l'article dépilatoire a été appliqué après le retrait de l'article dépilatoire avant l'application d'une composition de soin de la peau de post-traitement sur la zone du corps sur laquelle l'article dépilatoire a été appliqué.

10. Procédé d'élimination des poils indésirables de la surface du corps selon l'une quelconque des revendications précédentes, dans lequel le substrat souple a une rigidité dans l'intervalle allant de 5,00 g/cm à 0,08 g/cm, de préférence de 3,00 g/cm à 0,08 g/cm, plus préférablement de 1,80 g/cm à 0,10 g/cm, encore plus préférablement de 0,80 g/cm à 0,15 g/cm et même plus préférablement encore de 0,60 g/cm à 0,25 g/cm.

11. Procédé d'élimination des poils indésirables de la surface du corps selon l'une quelconque des revendications précédentes, dans lequel le substrat souple a un module sécant à une déformation de 2 % supérieur à 68,9 MPa (689,5 bar (10 000 psi)), plus préférablement supérieur à 137,9 MPa (1379,0 bar (20 000 psi)), encore plus préférablement supérieur à 206,8 MPa (2068,4 bar (30 000 psi)) et même plus préférablement encore supérieur à 275,8 MPa (2757,9 bar (40 000 psi)).

12. Procédé d'élimination des poils indésirables de la surface du corps selon l'une quelconque des revendications précédentes, dans lequel le substrat souple a une résistance nominale à la traction d'au moins 5 MPa, de préférence au moins 10 MPa, plus préférablement au moins 15 MPa et encore plus préférablement au moins 18 MPa.

13. Procédé d'élimination des poils indésirables de la surface du corps selon l'une quelconque des revendications précédentes, dans lequel l'article dépilatoire comprend au moins deux languettes pour le doigt positionnées sur des côtés essentiellement opposés de la région revêtue.

14. Procédé d'élimination des poils indésirables de la surface du corps selon l'une quelconque des revendications précédentes, dans lequel le substrat souple est imperméable à l'eau, comprend de préférence une feuille de plastique, plus préférablement une polyoléfine, encore plus préférablement un polyéthylène et même plus préférablement encore un polyéthylène à haute densité.

15. Procédé d'élimination des poils indésirables de la surface du corps selon l'une quelconque des revendications précédentes, dans lequel la composition dépilatoire est disposée sur le substrat souple en une quantité par surface unitaire de la région revêtue de 0,300 g/cm² à 0,001 g/cm², plus préférablement de 0,015 g/cm² à 0,003 g/cm², encore plus préférablement de 0,080 g/cm² à 0,005 g/cm² et même plus préférablement encore de 0,05 g/cm² à 0,005 g/cm².
